# EUROPEAN PATENT APPLICATION

(11) **EP 2 238 978 A1**
(43) Date of publication of application: **13.10.2010**
(21) Application number: 08866361.2
(22) Date of filing: 23.12.2008
(51) Int. Cl.: A61K 31/145, A61K 47/40, A61P 25/32, A61P 25/36

(54) **PHARMACEUTICAL COMPOSITIONS CONTAINING AN INCLUSION COMPLEX FORMED BY DISULFIRAM AND A CYCLODEXTRIN, WHICH CAN BE USED IN THE TREATMENT OF ALCOHOL AND COCAINE DEPENDENCE**

(30) Priority: 28.12.2007 CL 38772007
(71) Applicant: Universidad De Concepcion, Concepción (CL); Laboratorios Andromaco S.a., Santiago (CL); Abl Pharma Colombia S.a., Bogota (CO)
(72) Inventor: SEPULVEDA CARREÑO, Maria Jacqueline, Concepción (CL); VON PLESSING ROSSEL, Carlos Guillermo, Concepción (CL); MELLA GAJARDO, Fernando Pedro, Santiago (CL)
(74) Representative: Carvajal y Urquijo, Isabel
(86) International application number: PCT/IB2008/003696
(87) International publication number: WO 2009/083794

(57) **Abstract**

The present invention relates to a new pharmaceutical composition comprising a disulfiram complex (DSF) with a cyclodextrin (CD), its process for its production and use in the treatment of acohol and cocaine dependence.

## Description

### FIELD OF THE INVENTION

The present invention relates to a new pharmaceutical composition comprising a disulfiram complex (DSF) with a cyclodextrin (CD), its process for its production and use in the treatment of alcohol and cocaine dependence.

### BACKGROUND ART

Alcohol dependence is a multicausal pathology, so it must be approached by biopsychosocial treatment, including pharmacological support. There are several types of treatment for alcohol dependence, in which several pharmacological and/or phychotherapeutic tools are used, however they have in common the inconvenient of low adherence treatment which hinders the rehabilitation of the patient and their subsequent reintegration into society.

One of the most commonly used drugs in the treatment of alcohol dependence, is Disulfiram, (1-(diethylthiocarbamoyldisulfanyl)- N,N-diethyl-methanethioamide; CAS No. 97-77-8), molecular weight of 296,543 g / mol, Molecular formula C₁₀H₂₀N₂S₄, whose chemical structure corresponds to formula (I): 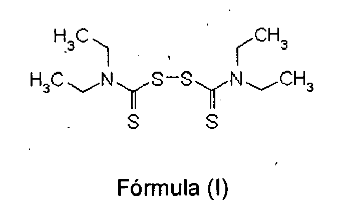 The first report on DSF and its potential applications in the treatment of alcohol dependence appears in 1948, by Jacobsen and Hald (Erik Jacobsen J. Hald, Larsen V. The sensitizing effect of tetraethyltiuram dysulphide (Antabuse) to ethyl alcohol, Acta Pharmacol et Toxicol, 4: 285-296, 1948). For years, this drug has been widely used as therapy for rejection of ethyl alcohol

The DSF produces alcohol hypersensitivity through the inhibition of the mechanism enzymatic that oxidizes the acetaldehyde to acetic acid, which occurs in the liver during the normal ethanol catabolism. Absorbed Ethyl alcohol is removed from the body primarily (> 90%) by oxidative mechanisms located in their great majority at the liver, and involving mainly the dehydrogenase alcohol enzyme and to a less proportion certain microsomal enzyme (microsomes oxidative alcohol). The first step in the alcohol metabolization, is oxidize the substrate to acetaldehyde through the system of the enzyme alcohol dehydrogenase (ADH), which uses the nicotinamide adenine dinucleotide (NAD+) as hydrogen acceptor. Most of the acetaldehyde continues oxidizing to acetic acid in the liver, by a family of isoforms of the enzyme aldehyde dehydrogenase (ALDH), which also uses NAD+ as hydrogen acceptor (Hardman, Joel G.; Limbird, Lee E. Goodman & Gilman, The Pharmacological Basis of Therapeutics, 9th edition, Vol. I, McGraw-Hill Interamericana Editores SA, Mexico, p. 417-418, 1996). Normally the ability to remove acetaldehyde by the ALDH exceeds the generation capacity of acetaldehyde by ethanol oxidation, so that circulating levels of acetaldehyde are quite low.

DSF competes with NAD+ ALDH enzyme producing an apparent irreversible inhibition of the enzyme activity (McEvoy, Gerald.AHFS Drug Information, 44th edition, American Society of Health-System Pharmacists Inc., Bethesda, USA 3630, 2002). It then produces an accumulation of acetaldehyde in the blood, which is responsible for the signs and symptoms, which have been named as "acetaldehyde syndrome" (ethanol-disulfiram reaction, RED), which appears within 10 minutes after the ethanol intake and that is mainly characterized by hot flushing, pulsating headache, dyspnoea, nausea, vomiting, sweating, hypotension, chest pain, dizziness and blurred vision. The duration of the syndrome varies from 30 minutes to several hours, then tiredness and sleep appears. This syndrome can be triggered even by 7 mL of ethanol on sensitive subjects. People undergoing treatment with DSF should be prevented that the use of shaving lotion or friction, such as medicines for cough syrups or sauces or fermented vinegar, can trigger the syndrome. The ethanol-disulfiram reaction can occur up to 1 or 2 weeks since the last taking the medicine.

DSF active metabolites causing clinically important effects on the body are: diethylthiocarbamate (DDC) and methyl diethylthiocarbamate (MeDDC).

DDC in addition to inhibit ALDH, inhibits dopamine-β-hydroxylase causing decline of epinephrine and norepinephrine in the heart, blood vessels and adrenal medulla, which would explain the hypotensive response and other cardiovascular reflexes of DSF *(*Nakako G, Holloway JE, Schackford, JS. Effects of disulfiram on the cardiovascular responses to ethanolin dogs and guinea pigs. Toxicology and applied pharmacology, 14 (3):439-446, 1969).

In the pharmaceutical market there are two types of drugs that are pellets and oral tablets, being the latter the most used in the therapy of alcohol dependence.

The success of the DSF oral use in the treatment of alcohol dependence depends on the patient's adherence to therapy by the appropriate period. This is because patients simply stop taking DSF and / or deceives the therapist leading to the abandonment of treatment. Any DSF therapy requires a support network composed of family and friends (O'Farrel TJ, Bayog RD. Antabuse contracts for married alcoholics and their spouses: A method to maintain Antabuse ingestion and decrease conflicts about drinking. J Subst Abuse Treat:3:1-8, 1986).

It has also been found benefit from the use of DSF in patients with cocaine dependence, with or without alcohol dependence comorbidities. (Carroll, K. M., Ziedonis, D., O'Malley, S. S., McCance-Katz, E., Gordon, L. and Rounsaville, B. J., Pharmacologic interventions for abusers of alcohol and cocaine: a pilot study of disulfiram versus naltrexone. American Journal on Addictions 2, 77-79, 1993*;* Carroll K. et al. Efficacy of disulfiram and cognitive behavior therapy in cocaine-dependent outpatients. Arch Gen Psychiatry, 61:, 264-72, 2004*.* Preti A. New developments in the- pharmacotherapy of cocaine abuse. Addict Biol, 12(2):133-51, 2007*;* y Kenna GA, Nielsen DM, Mello P, Schiesl A, Swift RM.Pharmacofherapy of dual substance abuse and dependence. CNS Drugs, 21(3):213-37, 2007*. Review).*

It has been discussed earlier that the disulfiram oral therapy has the disadvantage of the low rate of adherence to treatment and patients simply stop taking the drug. A radical strategy to prevent the abandonment of therapy was designed by Marie, who first introduced the subcutaneous implantation of 1 g of DSF (Marie, C. A Propos d'un Nouveau Mode de Traitement de l'Alcoolisme Chronique par Implantation de Disulfure de Tetraethyle-Thiourane. Thèse, Faculté de Médicine de l'Université de Paris, 1955). These implants or pellets have been used to improve the therapeutic use of disulfiram, however, because the therapeutic oral dose required are elevated (above 200 mg / day), it has been suggested that these pellets, usually 1 g does not reach effectives pharmacological levels and said effectiveness would be mainly placebo type *(*Wilson A., Davidson W, Blanchard R. Disulfiram implantation: A trial using placebo implants and two types of controls. J. Stud Alc, 41: 429-436, 1980).

Studies in animals have shown that the DSF can be effectively mobilized from the subcutaneous injection site if bioavailability is modified with oil. Stromme JH.; Eldjam L., Stensrud T., Distribution and chemical forms of diethyldithiocarbamate and tetraethyliuram disulfide (disulfiram) in mice in, relation to radioprotection. Biochem Pharmacol 15:287-297, 1966*; y* Phillips M., Gresser JG., Sustained-release characteristics of a new implantable formulation of disulfiram. J. Pharm Sci 73(12), 1718-1720, 1984), dissolved in polyethylene glycol (Phillips, M. Excretion of Carbon Disulfide in Breath Following Subcutaneous Injection of Disulfiram, Clinical Research, 1980; 28(3), 623 A*)* or water suspended (Phillips, Michael; Gresser, Joseph G. Sustained-Release Characetristics of a New Implantable Formulation of Disufliram, J. Pharm. Sci. Subst. Abuse, 1984; 73(12), 1718-1720). However, Phillips and Greenberg, (Phillips, M.; Greenberg, J. Dose-Ranging Study of Depot Disulfiram in Alcohol Abusers, Alcohol Cli. Exp- Res. 1992; 16(5): 964-967) showed that DSF water suspension, not act as a reservoir

Another type of implant has been devised by Chandia et al., (R. Chandia, E. Cid, Vallejos C., Avila JM. Clinical study of disulfiram in suspension for injection. A new altemative in the alcoholism treatment. Rev. Med. Chile, 118, 855-861, 1990*), who injected subcutaneously DSF micro suspended in a vehicle composed of propylene glycol*/*water.* The administration of 1 g by this system, resulted in intense local pain and a inflammatory response while maintaining levels of the active metabolite of disulfiram, diethyldithiocarbamate, higher than the traditional implant, at least for one month (Cid E. pharmacokinetic study of disulfiram administered in the form of injectable suspension. Theses to qualify for the title of Chemical-Pharmaceutical at the University of Chile, 1986).

Phillips and Greenberg, (M. Phillips, J. Greenberg Dose-Ranging Study of disulfiram depot in alcohol abusers. Alcohol Clin Exp Res. Vol 16 (5), 964-967, 1992) conducted a clinical trial with two pharmaceutical formulations of DSF deposit. In the first formulation, was administered from 1 to 3.5 g of DSF suspended in 5% of carboxymethylcellulose, which was difficult to injected because its high viscosity, also presenting low or no ethanol-disulfiram reaction, irritation and local pain, which demonstrated the ineffectiveness of this clinical formulation. In the second formulation viscosity was reduced administering from 60 to 90 mg/kg of DSF in 0.1% Tween 80, which helped ease administration and reduce a little the local trouble. However, this formulation should be administered with an attack dose of 15 mg/kg of oral DSF to obtain the expected clinical efficacy.

The inventors have surprisingly found pharmaceutical formulations that comprise an inclusion complex of CD with DSF, resolves many difficulties to get effective treatments

Document CN 1376463 A describes a pharmaceutical composition for topical ocular use which includes a CD and DSF, where the DSF has an improved solubility. The DSF and the CD was mixed in water and lyophilized. The final product is obtained formulating a lyophilized powder with water ready for eye aplication. This composition is an eye drops for treatment of cataracts and aims to increase the solubility of DSF, as the water solubility of this compound is a limiting factor to different types of physicochemical formulations, is about 0.02 mg/mL.

Wang S et al. (Wang S, Li D, Ito Y, Nabekura T, Bioavailability and anticataract effects of a topical ocular drug delivery system containing disulfiram and hydroxypropyl-beta-cyclodextrin on selenite-treated' rats. Current Eye Research. Vol. 29(1), 51-58, 2004) caried out a study of eye drops containing a high concentration of DSF and one CD.

The composition is designed to treat cataracts, and a high CDs concentration as Solubiliser is used to increase DSF solubility and get a pharmaceutical form suitable to use directly in the eye. In this paper we used a physical mixture of DSF with CD, not showing the formation of a complex but will only increase the solubility of the CD provided to the active principle.

The document EP 1574221 A describes injectable compositions of diclofenac complexed with one CD. This document does not suggest compositions for sustained release of active ingredient, but the CD is used to increase the solubility of active ingredient. The use of CD in the formulation of this document gives stability to the parenteral product.

According to background information delivered via the state of the art, it is clearly observed that it has not been completely successful in obtaining an effective treatment for alcohol dependency. The inventors of the present invention have developed new formulations that allow on one hand, a good adherence to treatment, which translates into less abandonment of therapy, and on the other hand, it has been able to avoid the inconvenience of parenteral or subcutaneous administration that cause pain and swelling in the inoculation site. In this way, with the modified release pharmaceutical forms developed in the present invention, adequate therapeutic levels are achieved, with excellent results in terms of its bioavailability and more important with a high clinical efficacy.

Therefore, an object of this invention is to provide dosage forms of modified release of DSF directed to overcome the constraints of the prior art mentioned above and which can be administered orally, intravenously, intramuscular and intradermal, among others.

### DETAILED DESCRIPTION OF THE INVENTION

The present invention describes new pharmaceutical compositions comprising an inclusion complex of CD with DSF as active ingredient. In the present invention hydroxypropyl-β-cyclodextrin (HP-β-CD) is used as a vehicle for the formation of a complex with DSF (hereinafter 'Complex DSF-CD'), which produces modifications to physical-chemical drug properties. This association allows the formulation of DSF pharmaceutical forms contributing to solve the present problems in treatment of alcohol dependence.

Surprisingly, the inventors have found pharmaceutical compositions comprising the association of DSF and HP-β-CD in the form of inclusion complex, improve the clinical effectiveness significantly increasing treatment adherence

On the other hand, the inventors have surprisingly found that association in the form of inclusion complex of DSF and HP-β-CD formulated in the presence of DSF without complexing (hereinafter 'free DSF'), also improves clinical effectiveness, increasing significantly treatment adherence

It is important to define here that in the context of this invention is to be understood that 'Physical Mixture' is a solid state mixture, with well-defined proportions of CD and DSF, and 'Binary Mixture' a mixture, in different proportions of free DSF and inclusion complex formed by t CD and DSF (DSF-CD Complex). Binary mixture will be defined according to the proportions of DSF free to DSF-CD Complex. Therefore, a Binary mixture 40:60 means it contains a proportion of free DSF to DSF-CD Complex 40:60.

The results obtained with the parenteral formulations surprisingly point to sustained release formulation even in the presence of CD, a recognized solubiliser agent.

In order to provide additional background regarding the inclusion complexes, these are defined as a form of chemical complex.in which a molecule or part thereof, the guest (in this case DSF active agent), is encapsulated or included within another molecule or structure made of such molecules, the host (in this case CD). The inclusion complex with CD is constituted by guest (DSF) and host (CD, in this particular case, the HP-β-CD). This asociation is characterized by the presence of weak intermolecular interactions, commonly called hydrophobic bonds similar to those found in various biological systems (enzyme-substrate, antibody-antigen, for example). One or more CD molecules may contain one or more guest molecules.

Surprisingly the proportion found by the inventors to form an inclusion complex of DSF CD to be used in a pharmaceutical formulation of modified release is DSF: CD from 1:1 to 1:2.

In the context of the invention "modified release" is defined as: (i) in the case of oral administration when at least the absorption rate, the Maximum Plasma Concentration (Cmax) and / or bioavailability (ABC), are higher than with the free DSF, and (ii) for parenteral administration, where at least plasma concentrations and / or the duration of these; are greater than that obtained with free DSF.

The interactions involved in the process of inclusion are weak in character and are attributed to Van der Waals forces, London dispersion forces, dipole-dipole interactions and hydrophobic interactions. In aqueous solution, water molecules are usually found in the internal cavity of cyclodextrins (interaction polarapolar), but since they are polar structures in nature, their presence is not thermodynamically favorable. When a guest is introduced within the CD, it displaces these water molecules. Both the guest and water molecules are thermodynamically favored. The result of the complexación is then, a system thermodynamically more favorable, the repulsion forces between water molecules whith high enthalpy within the CD and the repulsion forces between the apolar guest within the polar solution together with the interactions of weak type already mentioned, favor formation of the inclusion complex *(*Connors, K. The Stability of Cyclodextrin Complexes in Solution, Chemical Reviews, 97(5) :1325-1357, 1997; Szejtli, J., Introduction and General Overview of Cyclodextrin Chemistry, Chemical Reviews, 98(5): 1743-1753, 1998*;* Duchêne, D., New Trends in Cyclodextrins and Derivates, Editions de la Santé, Paris, 1992).

Over time, cyclodextrins have been chemically modified to improve the physical-chemical properties of the original compounds. That is how today we have the HP-β-CD, which comes from subsequent alkylation reactions of β-cyclodextrin, substitutions that were originally introduced in search of improving the solubility in water.

The pharmacology of HP-β-CD has been extensively studied by various authors. It should be kept in mind that it is a synthetic carbohydrate, not subject to active transport and can not pass through cell membranes as lipid barriers (due to its size and hydrophilicity), and as such, its distribution depends on the entry route to the body.

The HP-β-CD is presented to the amorphous state, has an excellent solubility, excellent stability, low tendency to crystallize and its safety and effectiveness have been established. Has shown excellent tolerance by different administration routes including intravenous, is not irritating to skin or eyes, and as the HP-β-CD is not toxic through parenteral via, is a compatible material to pharmaceutical design.

The absolute bioavailability is dose-dependent and minor than 0.5%; it presents limited distribution and renal clearance is equal to 80-90% of total clearance

The diversity of results in terms of DSF pharmacokinetics after parenteral administration is mainly due to drug insolubility and the impossibility to have a suspension to facilitate its administration, solubility and controlled release, with minimal impact on application area. That is why, it was thought the use of cyclodextrins due to their ability to interact with other molecules to form inclusion compounds, which can modify certain drugs and to give a series of features that allow better use with a maximum security For this reason, it was thought in the use of cyclodextrins due to their ability to interact with other molecules to form inclusion compounds, which can modify certain drugs and to give a series of drugs that allow better use with a maximum security (Szejtli J. Cyclodextrin News. Vol 9:159,1995).

This invention aims at solving the problems raised in the preceding paragraphs related to physical chemical properties, pharmacokinetic limitations, administration and DSF failure of therapy, by the design of a new pharmaceutical formulation of controlled release with therapeutic effect and that causes minimal damage at the injection (in the case of IM or SC administration), which comprises a DSF complex with CD (DSF-CD Complex).

DSF-CD Complex used in this invention has been developed by inventors at the University of Concepcion, Chile, and corresponds to an inclusion complex characterized by X-ray spectra, Differential Scanning Calorimetry (DSC), electron microscopy of Scanning (SEM), nuclear magnetic resonance (NMR) spectroscopy and infrared (IR).

To solve the technical problem described here, the inventors not only developed DSF-CD complex, but also had to investigate numerous formulation alternatives with various vehicles and excipients and different routes of administration to finally obtain the appropriate pharmaceutical compositions to solve the technical problem.

In a preferred realization it is taught pharmaceutical compositions comprising the Binary Mixture (which, as noted above corresponds to the complex mix of DSF with CD-free DSF) in a liquid dosage form, where the solvent is aqueous or oily.

In another preferred realization it is taught pharmaceutical compositions comprising the Binary Mixture in a solid dosage form.

In another preferred realization, it sets out the possible pharmaceutical compositions for the administration of the DSF-CD complex within which includes oral, bucal, intramuscular, subcutaneous, and dermal administration.

In a specific realization is described a form of parenteral administration modified release of a composition comprising the Binary Mixture and vehicles appropriate to formulate the compositions.

In another realization is described a specific form of oral administration (tablets) of modified release of a composition comprising the Binary Mixture and vehicles appropriate to formulate the compositions.

In another specific realization is described a form of subcutaneously solid adminisration (pellets) of modified release of a composition comprising the DSF-CD Complex and vehicles appropriate to formulate compositions.

In the preferred realizations of this invention, β-HP-CD is used. En las realizaciones preferidas de la presente invención, se utiliza la HP-β-CD.

In the pharmaceutical compositions comprising the complex DSF-CD it can be used different vehicles for proper suspension of the complex. These excipients are commonly used in the state of the art, and are selected from: water, glycerin, lecithin, 300 polyethylene glycol, propylene, miglyol, ethyl oleate, microcrystalline cellulose, magnesium stearate, Tween and mixtures thereof.

Within the excipients used for liquid dòsage forms are: miglyol, ethyl oleate, water, polyethylene glycol and propylene glycol.

The Miglyol is a medium chain triglyceride extracted as oil from the endosperm of coconut nucifera L. It is used in various pharmaceutical formulations including oral preparations, topical and parenteral. In parenteral formulations used for the preparation of emulsions, intravenous solutions and suspensions, being mainly investigated in total parenteral nutrition (TPN) in combination with long chain triglycerides.

The ethyl oleate is used mainly as a vehicle in parenteral preparations of intramuscular and subcutaneous use. Their characteristics are similar to almond oil and coconut oil, but it has the advantage of being less viscous than said oils and quickly absorbed by the body's tissues.

The inventors have surprisingly found that a formulation that contains the binary mixtures in a watery vehicle, has a clinical effect extended in time and thus must be administered only once a week or once every two weeks or once a month, depending on the dose used.

### DESCRIPTION OF THE FIGURES

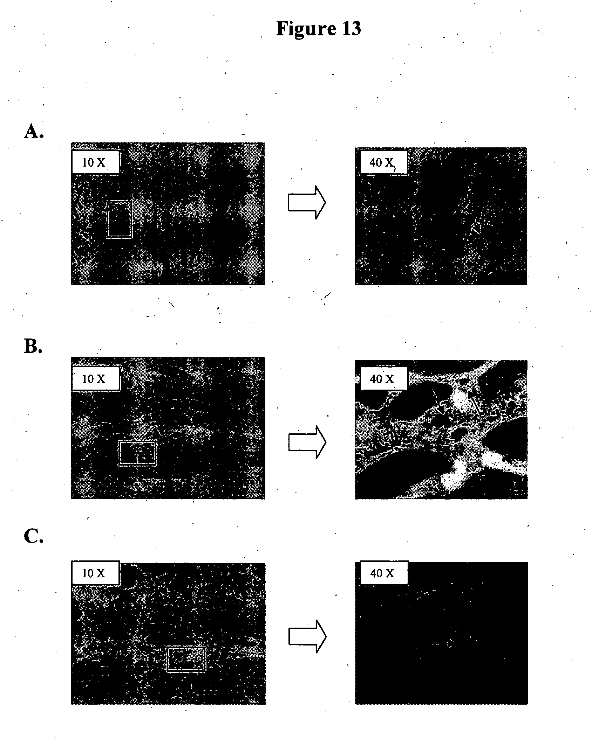

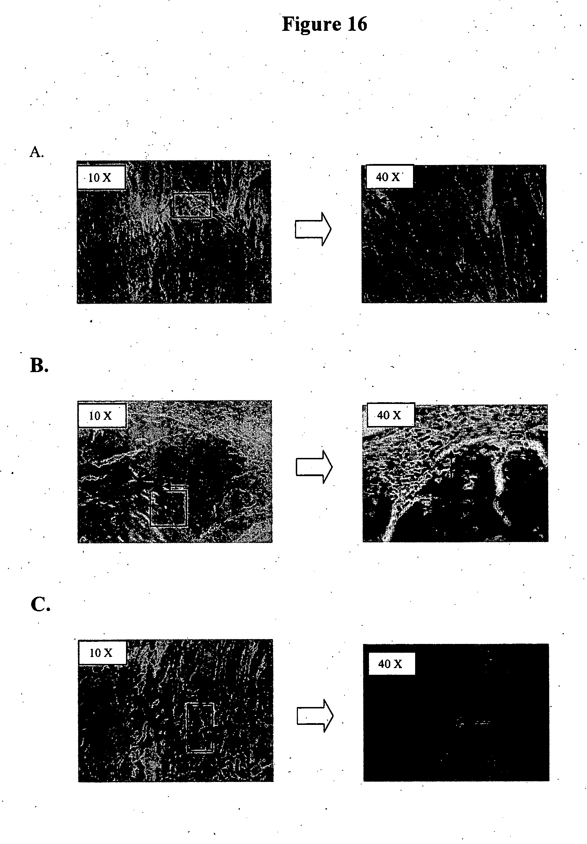

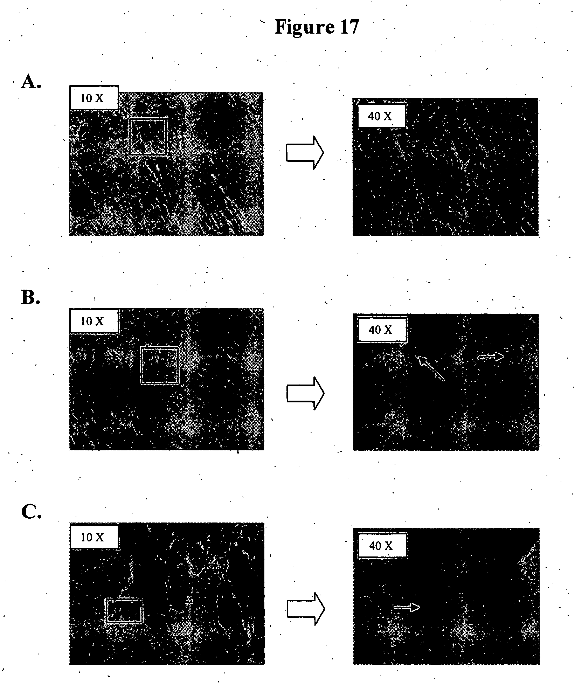

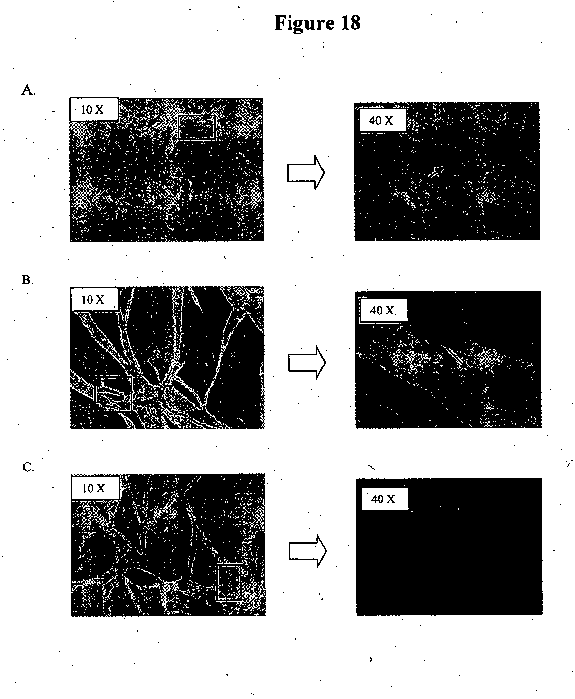

Figure 1: Kinetics of comparative dissolution of different formulations of tablets that contain the binary mixture.
Figure 2: Kinetics of comparative dissolution of the tablets containing the mixture Binary, for a formulation of the present invention (Example 10), compared with the tablets Antabus ® available on the market

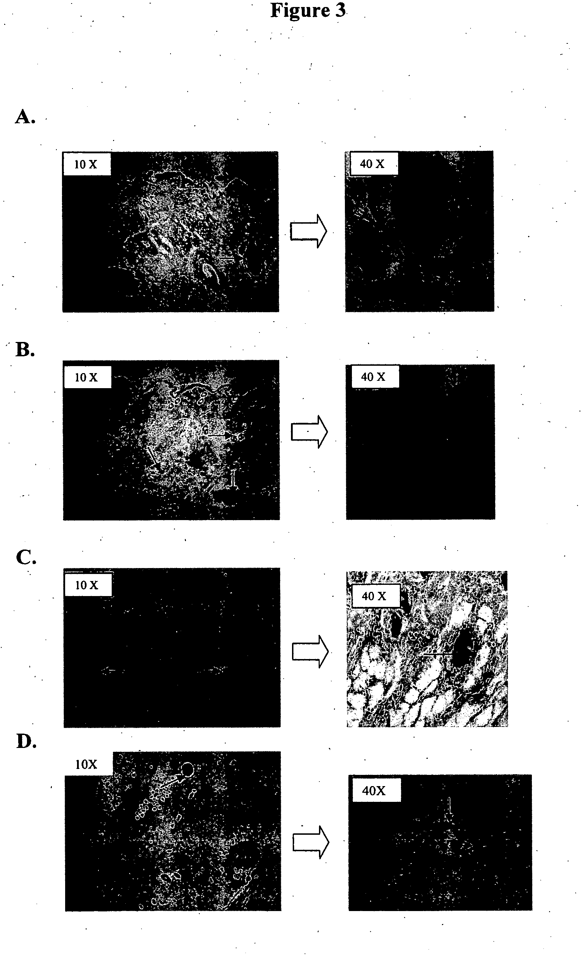

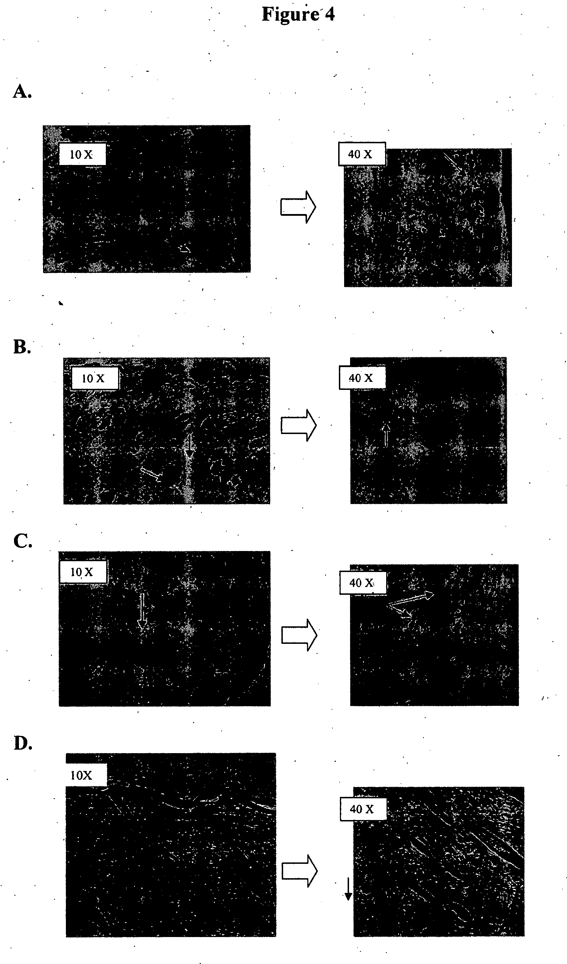

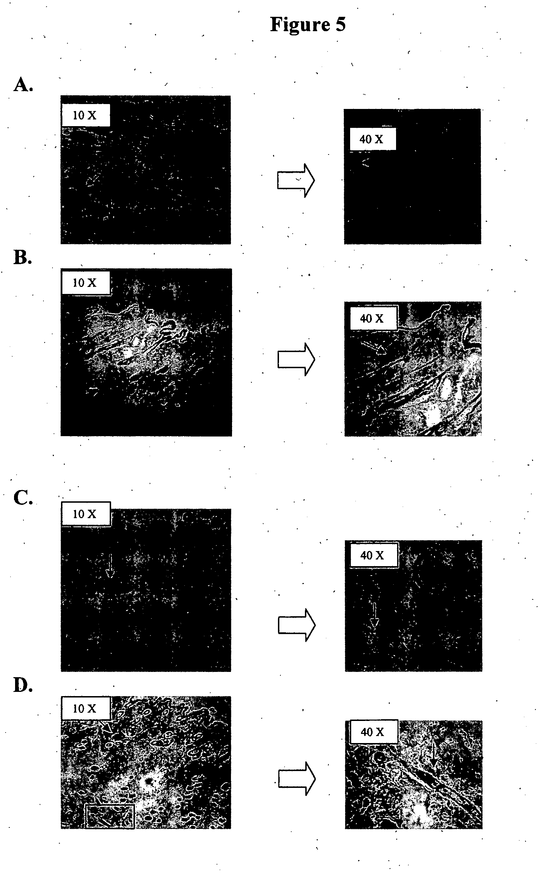

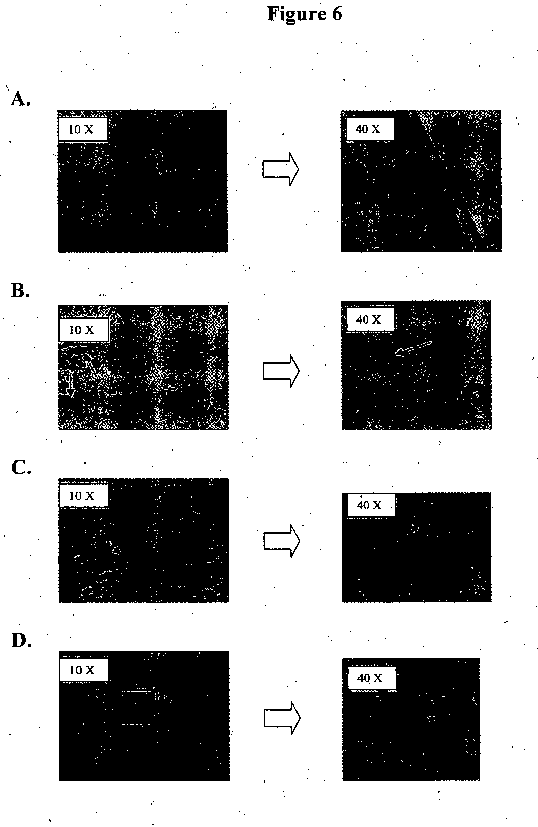

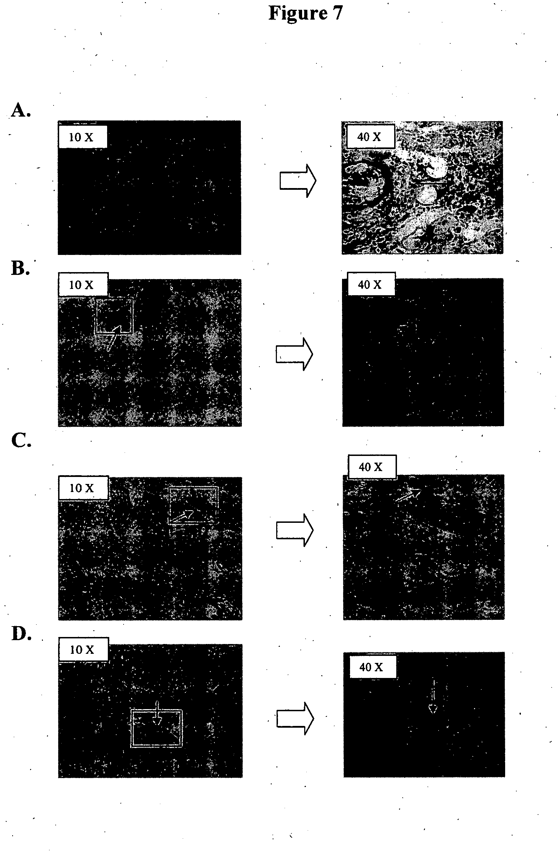

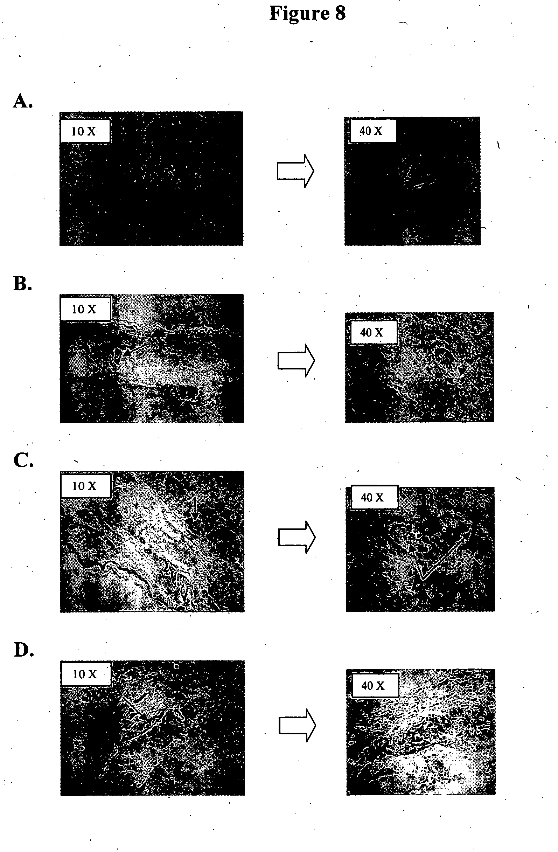

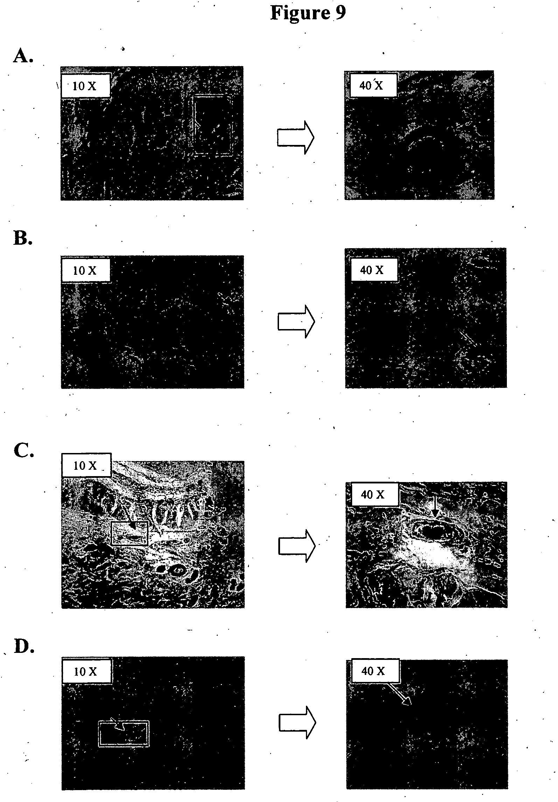

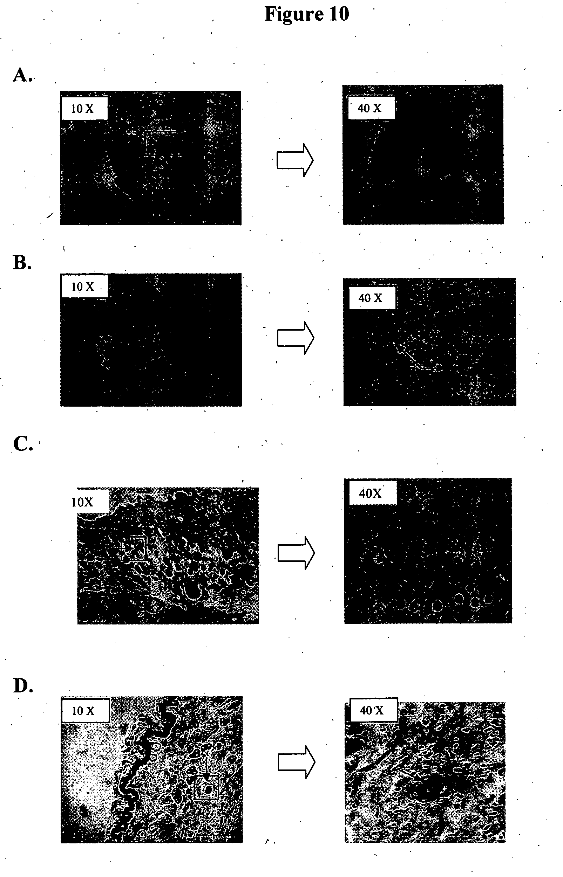

Figures 3 to 10: histological cuts of rat skin in the areas of subcutaneous administration (SC), 15 mg / kg of DSF to the form of DSF physiological serum (PS) (A); Complex DSF-CD in SF ( B), 25:75 Binary Mixture in SF (C) and control with SF (D). The histological were obtained at different times post-administration of each test. Figure 3 is up to 8 hours post-administration; Figure 4, to 24 hours post-administration; Figure 5, at 48 hours post-administration; Figure 6 to 72 hours post-administration; Figure 7, 96 hours post-administration; Figure 8, to 120 hours post-administration; Figure 9, to 144 hours post administration and Figure 10 corresponds to 168 hours post-administration.
Figure 11: histological cuts of rat muscle in the areas of intramuscular administration (IM), obtained at 24 and 168 hours post administration of 1.5 mg / kg miglyol (Control).
Figure 12: histological cuts of rat muscle in the areas of IM administration of 15 mg / kg of DSF to the form of Mixed Binary miglyol at 40:60 (A) and DSF to the form of binary mixtures at 40:60 oleate Ethyl (B). The histological cuts were obtained at 24 hours post-administration.
Figures 13, 14, 16-18: histological cuts of rat muscle in the areas of IM administration of 15 mg / kg of DSF to the form of Mixed Binary miglyol at 40:60 (A); DSF to the form of Binary Mixture Miglyol at 30:70 (B); DSF to the form of binary mixtures in 40:60 ethyl oleate (C).
   The histological cuts were obtained at different times post-administration of each test. Figure 13 corresponds to 48 hours post-administration, the picture 14 to 72 hours post-administration; Figure 16, to 120 hours post-administration; Figure 17, to 144 hours post-administration; Figure 18, 168 hours post-administration.

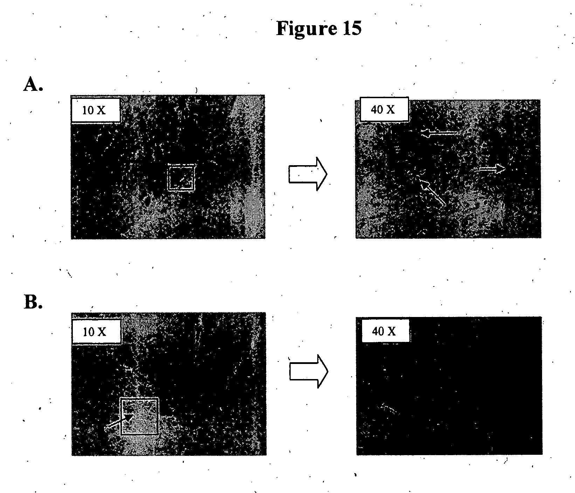

Figure 15: histological cuts in rat muscle in areas of IM administration of 15 mg / kg of DSF to the form of Mixed Binary miglyol at 40:60 (A) and DSF to the form of binary mixtures in 40:60 ethyl oleate (B). The histological were obtained at 96 hours post-administration.

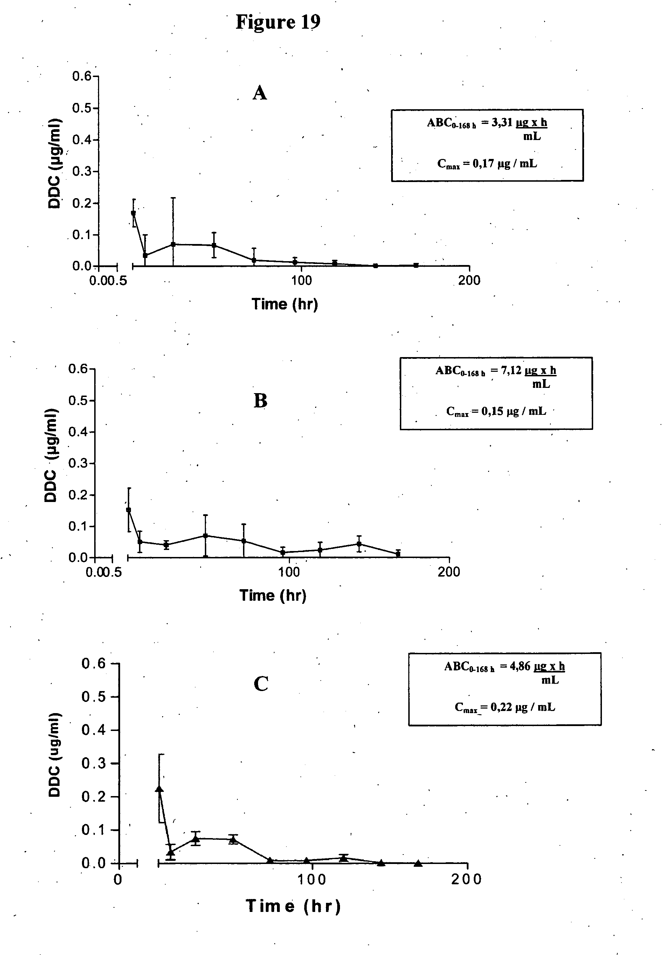

Figure 19: Effect of subcutaneous administration in rats, 15 mg / kg of DSF to the form of binary mixtures in water 25:75 (A); in miglyol (B) and (C) DSF free water on the concentrations Plasma total DDC in time.

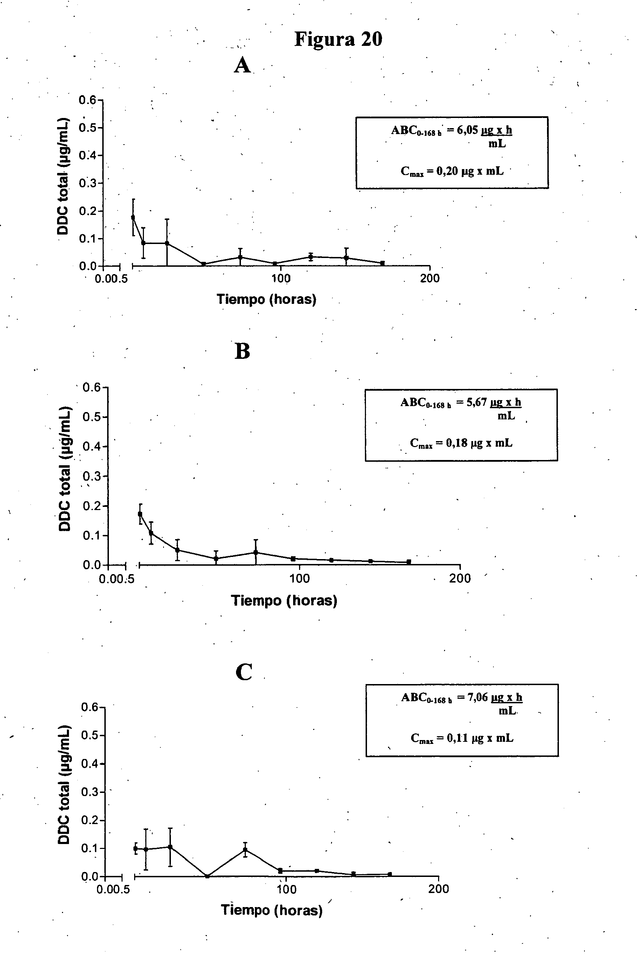

Figure 20: Effect of intramuscular administration in rats, 15 mg / kg of DSF to the form of Mixed Binary miglyol at 30:70 (A); the form of Mixed Binary miglyol at 40:60 (B); to form Mixing in binary 40:60 ethyl oleate (C) on the plasma concentrations of total DDC in time.

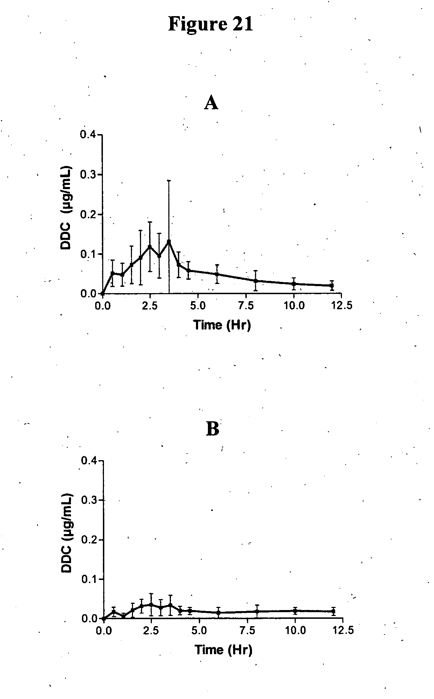

Figure 21: Effect of oral administration of 500 mg of DSF to the form of Binary Mixture 30:70 (A) and Antabus ® (B) on the plasma concentrations of total DDC in healthy volunteers.

### EXAMPLES OF PHARMACEUTICAL COMPOSITION WITH COMPLEX

### A. - Sample Pharmaceutical Compositions containing Complex DSF-CD vehicles in liquid formulations for parenteral (suspensions)

### EXAMPLE 1

| Binary mixture 30:70 | |
|---|---|
| DSF-CD complex | 2.54 g (equivalents to 450 mg of DSF) |
| DSF free | 1.050 g |
| Lecitine | 0.069 g |
| Propylparaben | 0.01 % |
| Methylparaben | 0.1 % |
| Vehicle citrate buffer | 3 mL |

The pharmaceutical compositions of Complex DSF-CD in liquid vehicles for parenteral formulations were prepared according to techniques known. It follows the protocol for the preparation of Example 1, which is comparable to procedures for the preparation of similar compositions mentioned later.

The preparation of the formulation of Example 1 was conducted in a sterile environment

In a mortar, the DSF and lecithin are joined. They were macerated to produce a uniform mixture of both compounds. It was solubilise propyl and methylparaben in a first part of the vehicle. Then it was added the DSF-CD complex with the remaining vehicle and get mixed up a paste free of lumps. Finally the suspension was packaged in glass ampoules previously sterilized.

### EXAMPLE 2:

| *Binary Mixture 40:60* | |
|---|---|
| DSF-CD complex | 2.66 g |
| DSF free | 0.53 g |
| Miglyol c.s.p. | 8.87 g |

### EXAMPLE 3:

| *Binary mixture 30:70* | | |
|---|---|---|
| DSF:CD complex | 1.94 | g |
| DSF free | 0.65 | g |
| Miglyol c.s.p | 8.27 | g |

### EXAMPLE 4:

| Binary mixture a *40:60* | | |
|---|---|---|
| DSF-CD Complex | 2.66 | g |
| DSF free | 0.53 | g |
| Ethyl oleate c.s.p | 8.85 | g |

### B. - Example of Pharmaceutical Compositions containing Complex DSF CD-solid formulations for oral administration

The pharmaceutical compositions of the DSF-CD Complex in vehicles for solid formulations in tablets were prepared according to known and techniques with conventional excipients for such purposes.

Different tablets were developed in which the ratio of the DSF and DSF Complex-CD was modified to obtain the optimal formulation. The following wording is a general formulation, detailed in Table 1, which sets the ranges of concentration for the main constituent of tablets weighing between 600 and 800 mg.

**Table 1: General formulation for solid formulations for oral administration.**

| | mg |
|---|---|
| DSF-CD complex | 360-640 |
| Disulfiram free | 120-320 |
| Avicel PH 101 | 0-90 |
| Aerosil | 0-15 |
| Ac-di- sol | 0-20 |
| Sdium starch glycolate | 0-60 |
| Dicalcium phosphate | 0-70 |
| Magnesium stearate | 1-10 |
| **Weight of tablet** | 600-800 |

Of all the formulations tested, 6 were chosen to carry out their profile formulations of dissolution, corresponding to 5 to 10 detailed examples in Table 2. These compositions presented times of desintegration compatible with oral administration.

**Table 2: Examples 5 to 10 tablets of the present invention (*).**

| | **Example 5** | **Example 6** | **Example 7** | **Example 8** | **Example 9** | **Example 10** |
|---|---|---|---|---|---|---|
| DSF-CD Complex | 424.2 | 424 | 424.2 | 424 | 424 | 424 |
| Disulfiram free | 175 | 175 | 175 | 174 | 175 | 175 |
| Avicel PH 101 | 0 | 0 | 0 | 80.5 | 29 | 29 |
| Aerosil | 0 | 0 | 0 | 2 | 0 | 5 |
| Ac-di- sol | 0 | 0 | 0 | 0 | 5 | 10 |
| Starch sodium glycolate | 49 | 44 | 49 | 14 | 0 | 0 |
| Dicalcium phosphate | 44,8 | 0 | 44,8 | 0 | 0 | 0 |
| Magnesium stearate | 7 | 7 | 7 | 3.5 | 2 | 2 |
| **Weight of the tablet** | 700 | 650 | 700 | 698 | 640 | 645 |
| disintegration time (min) | 8 | 13 | 12 | 8 | 9 | 8 |
| Hardness (Kp) | 10 | 5 | 3 | 11 | 4 | 3.5 |

| | | | | | | |
|---|---|---|---|---|---|---|
| (*) The quantities of all ingredients Examples of 5 to 10 are expressed in mg: | | | | | | |

Follows the general protocol of preparing the oral tablets.

### Protocol for development of tablets:

1. Sieve the DSF-CD Complex obtained at the University of Concepcion, Chile;
2. Preparing the solution of polyvinylpyrrolidone binder to 20% in alcohol solution;
3. Moisten the DSF with a binding solution;
4. Granulation itself, passing the moist mass through a sieve or mesh;
5. Dry the granules at 40° C for 24 hrs;
6. Homogenization of the particle size of the granules passing it through a sieve (size 195 m);
7. Mix with the rest of the excipients (disintegrate, thinner and finally the lubricant)
8. Compressing the final mix.

The tablets thus obtained were subjected to the basic controls of solid formulations such as, weight, hardness, disintegration and valuation of active ingredient.

It was performed the kinetics of dissolution of the tablets of Examples 5 to 10 (Table 2) under the conditions specified in the USP XXIV, using a computer disolutor Bahama St., Northridge with 6 cups, appliance 2: pallet method, a a speed of 50 revolutions per minute (rpm) for 60 min. and using a volume of 900 ml of water phase (water pH = 6.8, and 0.1 N HCl whatever) to 37 ± 0.5 degrees C.

Independent samples were taken during the period mentioned above with a sampling system fixed and automatic replenishment with manual volume.

Each formulation was included on the disolution means with agitation and temperature of the environment established. Samples were taken in mL of 5 intervals of 1, 3, 6, 10, 20, 30, 40, 50, 60 minutes with replacement of medium.

6 profiles were conducted with a dissolution n = 3

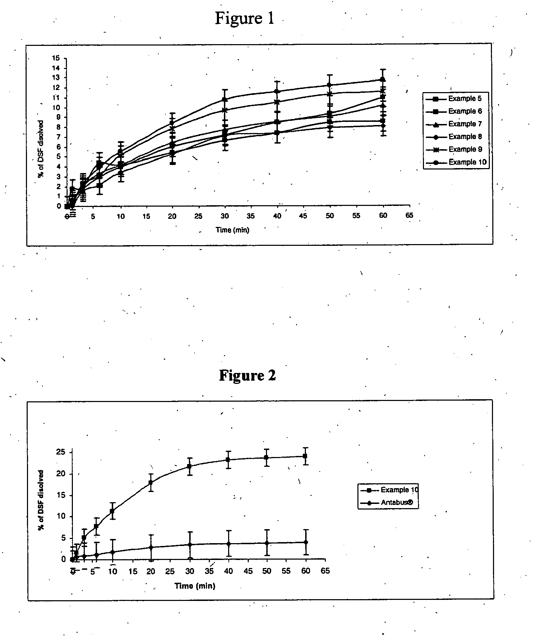

In Figure 1 it is shown the kinetics of dissolution of the 6 formulations that were tested and corresponding to tablets that contain the binary mixtures, according to the specifications set forth in Table 2 (Examples 5 to 10). Within 10 minutes all formulations showed more than 5% of DSF and dissolved more than 15% at 60 minutes, reaching approximately 25% for Example 10. Of all the formulations tested, the Example 10 had the best profile of dissolution, so for studies of bioavailability this formulation was selected.

As shown in Table' 2, the examples 5 to 10 presented times of disintegration appropriate for oral administration, but also showed a toughness that allows an appropriate handling throughout the process of packing, distribution, storage and management of tablets.

The formulation of Example 10 was compared with the tablets currently available in the market for the Antabus ®.

Figure 2 shows a comparison of kinetics of dissolution of the tablets Example 10 with the tablets of Antabus ®. It is noted that the tablets Antabus ® only reaches less than 5% of DSF dissolved after 60 minutes, in contrast with the tablets of the present invention that achieves up to 25% of DSF dissolved at this time, as well as giving a smaller dispersion in the results. In addition, one can see by comparing Figures 1 and 2, that all formulations of Figure 1, corresponding to formulations of the present invention (Examples 5 to 10) have a better profile of dissolution than Antabus ® in Figure 2.

That is, any chosen formulation of the present invention would be better than that existing in the market (Antabus ®).

### C.- Examples of Pharmaceutical Compositions containing DSF-CD Complex for solid formulations for subcutaneous administration (pellets).

This formulation is made by direct compression of the DSF-CD complex for the production of pellets, according to the conditions outlined in Example 11.

### EXAMPLE 11:

Each pellet contains:
DSF-CD complex equivalent to 25 mg of DSF

Dimensions of the pellets:
Diameter = 3 mm
Length = 7 mm

### PHARMACOLOGICAL EVALUATION OF THE COMPLEX PHARMACEUTICAL COMPOSITIONS.

### 1. Evaluation of the inflammatory intensity in the area of administration: histopathological study in rats

The histopathological evaluation was performed for subcutaneous (SC) and intramuscular (IM) injections of DSF free, in the form of CD-DSF Complex and in the form of binary mixture in ratios of 25:75 and 40:60. water, water with 15% glycerin, miglyol and ethyl oleate were used as excipients.

### Subcutaneous administration (SC)

15 mg / kg of free DSF, DSF Complex-CD, Mixed Binary 25:75 and physiological saline (SF) were administered subcutaneously (SC).

Comparative histological study between physiological saline and DSF in its various forms of administration are seen in Figures 3 to No. 10.

Figure 3 shows histological skin cuts of rats obtained 8 hours post-administration in different ways:
A. Free DSF injection: severe hyperemia was observed in the deep vascular plexus with bleeding in hypodermis
B. DSF-CD Complex injection: it was observed moderate and severe hyperemia in the superficial and deep vascular plexus respectively, with the presence of inflammatory cells;
C. Binary Mix Injection: shows slight presence of inflammatory cells and hyperemia of the deep vascular plexus;
D. SF injection: severe hyperemia was observed in both vascular plexus

Figure 4 shows histological skin cuts of rats, obtained 24 hours post-administration of the various administrations:
A. Injection of DSF free: severe hyperemia was observed in both plexus, abundant presence of inflammatory cells and hemorrhage in hypodermis;
B. Injection of DSF free: severe hyperemia was observed in both plexus, abundant presence of inflammatory cells and hemorrhage in hypodermis;
C. Binary Mix Injection: shows moderate hyperemia of deep vascular plexus and moderate presence of inflammatory cells;
D. SF Injection: shows slight desestructuration of collagen fibers.

Figure 5 shows histological skin of rats, obtained 48 hours post-administration of the various administrations:
A. Free DSF injection: it is observed severe desestructuration of the collagen fibers, hyperemia of deep vascular plexus and moderate bleeding in hypodermis;
B. DSF-CD Complex injection: shows moderate desestructuration of collagen fibers, hyperemia of deep vascular plexus and hemorrhage in hypodermis;
C. Binary Mix injection: shows moderate hyperemia of deep vascular plexus and mild presence of inflammatory cells.
D. SF injection: shows moderate presence of inflammatory cells and hyperemia of deep vascular plexus I

Figure 6 shows histological cuts of rat skin, obtained 72 hours post-administration of the various administrations:
A. Free DSF injection: moderate hyperemia was observed in both plexus, with moderate presence of inflammatory cells and hemorrhage in hypodermis;
B. DSF-CD Complex injection: shows moderate hyperemia of deep vascular plexus and severe presence of inflammatory cells;
C. Binary Mix injection: shows moderate hyperemia of deep vascular plexus and low presence of inflammatory cells;
D. SF injection: moderate hyperemia was observed with mild presence of inflammatory cells.

Figure 7 shows histological cuts of rats skin, obtained 96 hours post-administration of the various administrations:
A. Free DSF injection: it is observed moderate hyperemia of deep vascular plexus, with hemorrhage in hypodermis and severe presence of inflammatory cells;
B. DSF-CD Complex injection: shows moderate hyperemia of deep vascular plexus with mild presence of inflammatory cells, neutrophils in diapédesis and edema in dermis;
C. Binary Mix injection: shows moderate presence of inflammatory cells and hyperemia of the deep vascular plexus. With slight hemorrhage in hypodermis;
D. SF injection: shows mild hyperemia of the deep vascular plexus.

Figure 8 shows histological cuts of rats skin, obtained 120 hours post-administration of the various administrations:
A. Free DSF injection: it is observed moderate hyperemia of deep vascular plexus, with slight presence of inflammatory cells and desestructuration of collagen fibers.
B. DSF-CD Complex injection: shows mild hyperemia of the deep vascular plexus, presence of inflammatory cells and hemorrhage in hypodermis;
C. Binary Mix injection: shows slight hyperemia of deep vascular plexus and low presence of inflammatory cells;
D. SF injection:shows mild hyperemia of the deep vascular plexus, with low presence of fibroblasts.

Figure 9 shows histological cuts of rats skin, obtained 144 hours post-administration of the various administrations:
A. Free DSF injection: it is noted moderate hyperemia of deep vascular plexus and low presence of inflammatory cells;
B. DSF-CD Complex injection: low hyperemia was observed in both plexus and moderate presence of inflammatory cells;
C. Binary Mix injection: shows moderate hyperemia of deep vascular plexus with low presence of inflammatory cells;
D. SF injection: shows mild hyperemia of the deep vascular plexus.

Figure 10 shows histological cuts of rats skin, obtained 168 hours post-administration of the various administrations:
A. Free DSF injection: it is observed moderate hyperemia of deep vascular plexus;
B. DSF-CD Complex injection: shows severe hyperemia of deep vascular plexus with moderate hemorrhage in subcutis and abundant presence of fibroblasts;
C. Binary Mix injection: shows mild hyperemia of the deep vascular plexus;
D. SF injection: it is observed hyperemia of the deep and superficial vascular plexus.A.

In analyzing the results of the histopathological lesions with the administration of free DSF versus DSF-CD Complex and Disulfiram in the form of binary mixtures, it is shown that the DSF produces a desestructuration of the collagen fibers that progressed from mild, moderate to severe, at 8, 24 and 48 hours from inoculation, damage which declined to moderate in the case of DSF complex and to mild in the case of DSF to the form of binary mixtures.

The presence of inflammatory cells, superficial vascular plexus hyperemia and hemorrhage in subcutis went from moderate to severe in controls between 24 to 96 hours in the case of free DSF, unlike what happened in the DSF complex where the large presence of inflammatory cells occurred early in the control of the 8 hours persisting with a moderate character until 72 hours. Hyperemia of superficial vascular plexus was mild and short-lived during the first 24 hours, while the edema and hemorrhage in subcutis decreased to moderate in the controls at 24 and 72 hours.

In the case of DSF in the form of binary mixtures it was observed a significant decrease in lesions where there was a slight agglomeration of inflammatory cells and hemorrhage in subcutis. Hyperemia of deep vascular plexus in the case of free DSF was severe in biopsies of 8 and 24 hours and mild to moderate in subsequent checks, Another case was the cyclical behavior of this lesion in the rats treated with complex DSF , where the severity of the lesion was observed at the beginning and end of the study (8-168 hours), being mild to moderate in the intermediate controls. In the case of DSF in the form of binary mixtures it was observed that this injury as a mild to moderate over all the controls of the study.

In conclusion, the SC administration of DSF produces severe Histopathological lesions which are attenuated with the administration of DSF in the form of complex or Binary mixture.

### Intramuscular administration.

The histopathological lesions in the intramuscular administration in rat muscle were evaluated, obtained post-administration of 15 mg / kg of DSF in the form of binary mixtures in the proportions of 30:70 and 40:60 at 500 □ L or 500 of miglyol □ L of ethyl oleate.

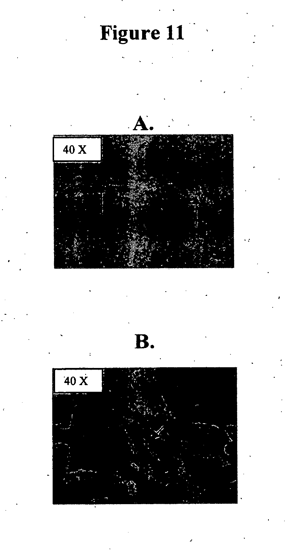

Figure 11 shows a histological cut in rat muscle, in areas of administration IM, obtained at 24 and 168 hours post-administration of 1.5 mg / kg miglyol as a control.
A. 24 hrs. post-administration: mild hyperemia was observed in perimysium, with diapédesis of neutrophils and lymphocytes, with little presence of inflammatory cells
B. 168 hrs. post-administration, the muscle looks normal, with just a little hyperemia of the vessels are located in the perimysium, with slight thickening of the collagen fibers in endomysium and perimysium. 1

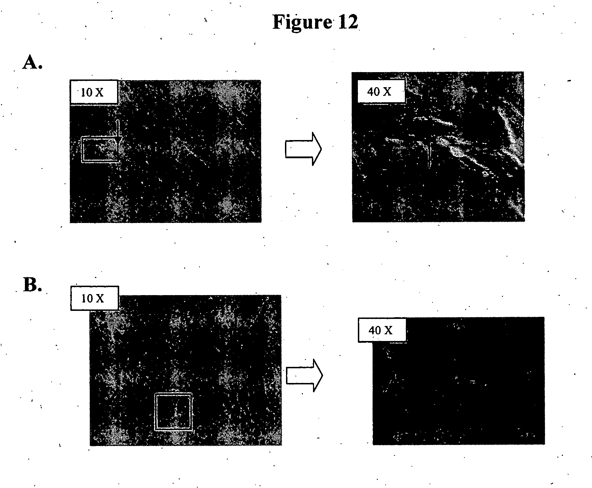

Figure 12 shows a histological cut obtained 24 hr post-administration:
A. Figure 12 shows a cut histological obtained 24 hr post-administration:
B. Binary mixture (40:60) in ethyl oleate: shows small areas of desestructuration in muscle fibers. Linfoplasmocitaria population in moderate amount, in perimysium

Figure 13 shows a histological cut obtained 48 hours post-administration:
A. Binary mixture (40:60) in miglyol: shows moderate amount of inflammatory cells of linfoplasmocitario type and little disturbance of muscle cells;
B. Binary mixture (30:70) in miglyol: shows moderate amount of inflammatory cells of linfoplasmocitario type in epimisio;
C. Binary mixture (40:60) in ethyl oleate: shows large areas of desestructuration of muscle fibers with loss of nuclei and breaking of fibers. abundant inflammatory Population ,in epimisio and perimysium. With areas of hemorrhage in perimysium

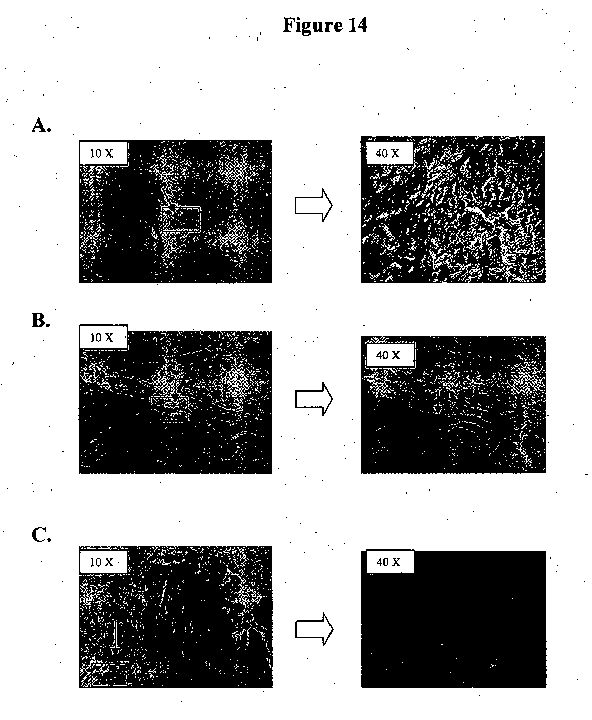

Figure 14 shows a histological cut obtained 72 hours post-administration:
A. Binary mixture (40:60) in miglyol: moderate to severe hyperemia was observed in epimisio vessels, with abundant amount of inflammatory cells, of linfoplasmocitario type and a moderate to severe increase of fibroblast and satellite cells.
B. Binary mixture (30:70) in miglyol: moderate to severe hyperemia was observed in epimisio vessels, with moderate to abundant linfoplasmocitaria population, in epimisio;
C. Binary mixture (40:60) in ethyl oleate: desestructuration is observed in muscle fibers, with loss of nuclei and breaking of fibers. mild Hyperemia in perimysium mild, with moderate inflammatory population, in perimysium and epimisio.

Figure 15 shows a histological cut obtained 96 hours post-administration:
A. Binary mixture (40:60) in miglyol: it is observed normal muscle fiber structure and low to moderate inflammatory population, in epimisio;
B. Binary mixture (40:60) in ethyl oleate: shows small areas of desestructured muscle fibers, moderate presence of linfoplasmocitarias cells in epimisio, and moderate to severe hyperemia, in perimysium and epimisio.

Figure 16 shows a histological cut obtained 120 hours post-administration:
A. Binary mixture (40:60) in miglyol: it is observed areas of fibers with loss of nuclei. Hyperemia mild to moderate. Abundant amount of inflammatory cells of linfoplasmocitario type. Moderate increase of population of fibroblast and satellites cells;
B. Binary mixture (30:70) in miglyol: normal muscle fibers were observed and moderate linfoplasmocitaria presence.in epimisio;
C. Binary mixture (40:60) in ethyl oleate: linfoplasmocitaria inflammatory cells were observed in median amount in perimysium, with regular hipermia in the same area of connective tissue.

Figure 17 shows a histological cut obtained 144 hours post-administration:
A. Binary mixture (40:60) in miglyol: shows slight alteration of the muscle fibers, with loss of nuclei. Mild hyperemia in vessels of perimysium. Moderate amount of inflammatory cells of linfoplasmocitario type;
B. Binary mixture (30:70) in miglyol: it is observed small areas desestructuration of muscle fibers, with loss of nuclei, moderate linfoplasmocitaria population, in epimisio;
C. Binary mixture (40:60) in ethyl oleate, there was normal structure of muscle fibers, with few cell types linfoplasmocitario in epimisio.

Figure 18 shows a histological cut obtained 168 hours post-administration:
A. Binary mixture (40:60) in miglyol: it is observed a slight deterioration in the muscle fibers, with loss of nuclei and sparse amount of inflammatory cells of linfoplasmocitario type in epimisio;
B. Binary mixture (30:70) in miglyol: there are small areas of desestructuration of muscle fibers , with loss of nuclei. moderate to abundant inflammatory population, in,epimisio;
C. Binary mixture (40:60) in ethyl oleate: it is observed structure of muscle fiber normal and a moderate to abundant inflammatory population.

From the results it is concluded that binary mixtures are compatible with the route of administration IM, as it produces minimal alterations in the tissue after its administration.

### PHARMACOKINETICS STUDIES ESTUDIOS FARMACOCINETICOS

In addition to the histopathological studies in rats pharmacokinetic studies were conducted that measured the plasma concentrations of metabolites of DSF (DDC and MeDDC) after administration SC (Figure 19) and / or IM (Figure 20), 15 mg / kg weight of DSF to the form of binary mixtures.

It was quantified the plasma concentration of total DDC that corresponds to the sum of the metabolites and MeDDC DDC, and it was used as a parameter for the analysis of the results shown below.

Figure 19 shows the effect of the SC administration of DSF in the form of binary mixtures (25:75) using water as a solvent, on the plasma concentrations of total DDC in time. The Cmax of 0.17 mg / mL was achieved after one hour of administration and detectable concentrations of total DDC were mantained even up to 168 hours post-administration, obtaining an area under the curve (AUC) of 3.31 (g xh) / mL.

Figure 19 B corresponds to the effect of SC administration of DSF in the form of a binary mixture (25:75) using miglyol as a solvent on plasma concentrations of total DDC over time. The Cmax of 0.15 mg / mL was also reached after one hour of administration and, as before, detectable concentrations of total DDC were mantained even up to 168 hours post-administration, obtaining an area under the curve (ABC) 7.12 (g xh) / mL.

Figure 19 C corresponds to the effect of SC administration of free DSF using water as a solvent, on the plasma concentrations of total DDC over time. The Cmax of 0.22 mg / mL was also reached after one hour of administration and significantly higher concentrations of DDC total were mantained during the first 144 hours post-administration, obtaining an area under the curve (AUC) of 4.86 (g xh) / mL. At 168 hours post-administration plasma levels of active metabolites were not detected.

In Figure 19 it is observed that with the SC administration of the formulations that contain the binary mixtures (Figure 19 A and 19 B) better results were obtained, since although in all cases bioavailability (ABC) was not significantly increased; detectable concentrations of the active metabolites (DDC total) were obtained for more time compared to the SC administration of free DSF (Figure 19 C), which suggests a longer therapeutic effect for the formulations of the invention. Even though a top ABC was not reached since the formulation of Figure 19 is only a suspension of binary mixtures in water, without any adjuvant to improve the characteristics of the formulation.

Figure 20 shows the effect of the IM administration of the Binary Mixture (30:70) using miglyol as a solvent, on plasma concentrations of total DDC over time. The Cmax of 0.17 mg / mL was reached after one hour of the administration, same as when administered via SC (Figure 19). At 168 hours post-administration, still there were detectable concentrations of total DDC, obtaining an area under the curve (AUC) of 6.05 (g xh) / mL.

Like the picture above, the picture 20 B shows the effect of the IM administration of the Binary mixture in miglyol but in a ratio of 40:60, on the plasma concentrations of total DDC over time. In this case, the Cmax was 0.17 mg / mL after one hour of administration and detectable levels of DDC total were mantained until 168 hours post-administration, with an area under the curve (AUC) 5.67 (g xh) / mL.

In Figure 20 C it is observed the effect on plasma concentrations of total DDC, after IM administration of the Binary Mixture (40:60), using ethyl oleate as a solvent. A lower Cmax, 0.10 mg / mL was reached after 24 hours of administration. In addition, with this formulation in ethyl oleate, plasma levels of DDC were more erratic, because at 48 hours there was no active metabolites in plasma but at longer times they were detectable, reaching an area under the curve (ABC ) Increased from 7.06 (g xh) / mL, compared with the same mixture of Binary but miglyol as a solvent (Figure 20B).

With all the proportions of Binary Mixtures and with the different vehicles used, with the IM administration were obtained areas under the curve greater than with an SC administration. This shows that the IM way is clinically superior.

### 2. CLINICAL STUDIES IN HUMAN

### A. BIOAVAILABILITY STUDIES IN HEALTHY VOLUNTEERS

We performed, a clinical protocol to study the bioavailability of DSF tablets in healthy volunteers, using the formulation of Example 10 of the present invention.

The investigated Products were
Output reference: Antabus ® Tablets 500 mg. Laboratories Farmoquimica Pacific (Formulation A).
Test Product: Tablets of 250 mg. Faculty of Pharmacy, University of Concepcion, Chile, for example 10 of the present invention (Formulation B).

### Experimental design:

The design is to study the bioavailability of two formulations: a reference (Formulation A) and a product of the invention (Formulation B) in two periods and two sequences.

In the first period, half the subjects received the Formulation A (1 tablet of Antabus ® 500 mg) in the second period they received the Formulation B (2 tablets of the innovative formulation of 250 mg ea). For the other half of the volunteer subjects, the situation is exactly the opposite.

### SUBJECT:

The population of male subjects for studies of bioavailability should be as uniform as possible.

Number of subjects: 8 volunteers.

### Analytical methodology:

It was used an analytical method based on high-performance liquid chromatography (HPLC) with UV detection for the determination of DDC and MeDDC in plasma of the volunteers, previously validated for pharmacokinetic studies.

The results of Figure 21, show that with the administration of the formulation of the invention corresponding to Example 10 (Figure 21) it was obtained areas under the curve (AUC) significantly higher than those obtained with the formulation of reference (Antabus ®) (Figure 21 B).

The ABC obtained with the tablets of the present invention is 0617 (g xh) / mL, while the same parameter for the tablets for the formulation of reference was 0227 (g xh) / mL.

This indicates that with the formulation that includes the binary mixtures achieve plasma levels of total DDC higher than with the tablets of Antabus to equal doses administered, allowing a viable form in the lower doses to achieve the same therapeutic effect

### B. CLINICAL STUDIES ON EFFECTIVENESS IN PATIENTS WITH. ALCOHOL DEPENDENCE

A liquid formulation was tested for IM administration of the present invention (Example 1) in patients of the Drug Addiction Unit Complex and Dual Pathology Service Psychiatric Hospital Guillermo Grant Benavente, establishment that meets the basic requirements of infrastructure and adequate personnel to Phase studies.

The study involved patients with alcohol dependence, who prior to the start of the study signed an informed consent. The criteria were dependent on alcohol according to ICD-10, of the International Classification of Diseases, 10th version of the World Health Organization.

To 29 patients it was administered via IM the formulation of Example 1 of the present invention, in a dose of 1 g once a month.

Other 19 patients received via oral the formulation of reference Antabus ® at a dose of 500 mg per day.

To determine the clinical effect achieved it was measured:
- Levels of Abstinence and Adhesion after 3 months of treatment;
- Levels of Abstinence and Adherence after 6 months of treatment.

Parenteral administration was well tolerated and there was no foreign body reaction.

Next, the results in relation to the measured parameters in the patients treated: Abstinence and Adherence to therapy.

**Table 3. Clinical effectiveness of an IM formulation according to the present invention and Antabus ® in the treatment for alcohol dependency.**

| Treatment with DSF | 3 months of treatment | | | 6 months of treatment | | |
|---|---|---|---|---|---|---|
| | N° de Pacientes | Abstinencia (%) | Adherencia (%) | N° de Pacientes | Abstinencia (%) | Adherencia (%) |
| Parenteral (Example 1) | 29 | 98,3 | 69,43 | 29 | 98,9 | 57 |
| Oral (Antabus®) | 19 | 83,3 | 62,6 | 19 | 88,3 | 15,6 |

The Abstinence is related to the number of days that the patient does not consume alcohol.

Adhesion corresponds to the actual number of therapeutic activities for the participation of the patient regarding their commitment to the initial treatment.

With the formulation of the invention it is obtained a better clinical response, with greater Abstinence and adhesion, compared with conventional therapy (Antabus ®).

These results confirm that with an IM administration once a month, better clinical results were achieved than with a daily oral administration of the tablets currently on the market.

## Claims

1. A pharmaceutical composition wherein said composition comprises an inclusion complex that includes disulfiram (DSF) and cyclodextrin (CD), optionally free or non-complexed DSF and one or more pharmaceutically acceptable vehicles.

2. A pharmaceutical composition according to claim 1 wherein said CD is hydroxypropil-β-cyclodextrin.

3. A pharmaceutical composition according to claim 1 wherein said composition comprises free or non-complexed DSF.

4. A pharmaceutical composition according to claim 1 wherein said composition is contained in a solid pharmaceutical form.

5. A pharmaceutical composition according to claim 4 wherein said solid pharmaceutical composition is administered orally or subcutaneously.

6. A pharmaceutical composition according to claim 1 wherein said composition is contained in a liquid pharmaceutical form.

7. A pharmaceutical composition according to claim 6 wherein said liquid pharmaceutical form is administered orally or parenterally.

8. A pharmaceutical composition according to claim 7 wherein said liquid pharmaceutical composition is administered subcutaneously or intramuscularly.

9. A pharmaceutical composition according to claim 3 wherein the ratio between free DSF and complexed DSF ranges from 20:80 to 40:60.

10. A pharmaceutical composition according to claim 9 wherein the ratio between free DSF and complexed DSF is 25:75.

11. A pharmaceutical composition according to claim 9 wherein the ratio between free DSF and complexed DSF is 30:70.

12. A pharmaceutical composition according to claim 9 wherein the ratio between free DSF and complexed DSF is 40:60.

13. A pharmaceutical composition according to claims 1 and 7 wherein the pharmaceutically acceptable vehicle for liquid pharmaceutical forms is selected from the group consisting of water, miglyol and ethyl oleate.

14. Use of the pharmaceutical composition according to any of the claims 1 to 13 wherein said composition is useful to prepare a medicament to treat alcohol dependency.

15. Use of the pharmaceutical composition according to any of the claims 1 to 13 wherein said composition is useful to prepare a medicament to treat cocaine
